# EUROPEAN PATENT SPECIFICATION

(11) **EP 1 948 305 B1**
(45) Date of publication and mention of the grant of the patent: **07.10.2015**
(21) Application number: 06828936.2
(22) Date of filing: 07.11.2006
(51) Int. Cl.: A61N 1/39, A61N 1/05, A61N 1/04

(54) **DEFIBRILLATION PADDLE STRUCTURE**
DEFIBRILLATIONS-PADDELSTRUKTUR
STRUCTURE D'ÉLECTRODE DE DÉFIBRILLATION

(30) Priority: 07.11.2005 US 267428
(43) Date of publication of application: 30.07.2008
(73) Proprietor: Epstein, Stephen, T., Newtown PA 18940 (US); Martens, André, 3350 Linter (BE)
(72) Inventor: Epstein, Stephen, T., Newtown PA 18940 (US); Martens, André, 3350 Linter (BE)
(74) Representative: D'Halleweyn, Nele Veerle Trees Gertrudis
(86) International application number: PCT/EP2006/010641
(87) International publication number: WO 2007/051648

(56) References cited:
- WO-A-00/22981
- WO-A-97/28844
- GB-A- 2 386 559
- US-A1- 2002 198 583
- US-A1- 2003 191 501

## Description

The invention relates to a defibrillation paddle assembly, and a defibrillation system. In general, the present invention relates to defibrillation paddles. More particularly, the present invention relates to defibrillation paddles that are used internally within the body and directly contact the tissue of the heart.

US 2003/0191501 discloses to use a pair of defibrillation paddles where an electric discharge is passed from on spoon electrode through the heart to another spoon electrode. The paddles are pre-shaped, and disposable.

It is known that a heart muscle that has stopped or is beating erratically can sometimes be caused to beat normally by passing an electric charge to the heart. The science of where, when and how to apply an electrical charge to the heart has been evolving for many years.

Defibrillators are machines that are specifically designed to pass an electrical charge into a patient's heart. In the field of defibrillators, there are non-intrusive defibrillators and intrusive defibrillators. Non-intrusive defibrillators have electrodes that attach to the skin of a patient. The non-intrusive defibrillators pass an electric charge through the body from one external point to another. Such non-intrusive defibrillators are used by rescue workers, paramedics and the like to revive a person whose heart may have stopped.

Since non-intrusive defibrillators pass electricity through the body, hoping to effect the heart, large electrical charges are used. These charges often cause the skin of the patient to burn at the areas where the defibrillator is attached to the body and where electricity passes into and out of the patient's body.

Intrusive defibrillators are used by physicians primarily in the operating room of hospitals. During many surgical procedures, a patient's heart may be temporarily stopped. In such situations, the patient's blood flow is transferred to an external pump. Once the surgical procedure is complete, a defibrillator is commonly used to restart the heart. When the heart muscle itself is exposed, the paddle terminals of the defibrillator are touched directly to the heart muscle. A small jolt of electricity is then passed through the tissue of the heart muscle to restart the heart's beat. Since the electricity is being applied directly to the heart muscle, smaller charges of electricity are used. However, even these smaller charges of electricity can result in some heart muscle tissue becoming burned in the areas of contact with the defibrillator's paddles.

One way to reduce the potential of damage to the heart muscle is to decrease the surface area of the heart that is physically contacted during a defibrillation attempt. In the prior art, defibrillator systems have been made that use only one paddle. In such systems, a patient is laid upon a conductive pad. A single paddle is provided. Both the single paddle and the conductive pad are connected to the defibrillator. The single paddle is then touched to the tissue of the heart. Electricity passes through the heart, through the back of the body and to the conductive pad. Since only one paddle is used, the area of the heart that directly contacts the paddle is reduced in half. Such prior art defibrillation systems are exemplified by Japanese Reference No JP 2001 218854, entitled Defibrillating Electrode And Defibrillation System.

A key to avoiding tissue damage made by a defibrillator paddle is to have uniform contact between the paddle and the tissue of the heart muscle. With prior art defibrillation systems, the paddle of the defibrillator is a fixed structure.

Depending upon the reactions of the heart muscle, a doctor may decide to shock the heart at a specific spot. That location may be on the top of the heart or along one of the sides of the heart. However, it is often difficult to maneuver the defibrillator paddle to that portion of the heart while still maintaining a flush contact between the defibrillator paddle and the tissue of the heart. If there is not a flush contact, the odds greatly increase that the defibrillator paddle may cause an electrical burn upon the heart. Furthermore, without a flush contact, the defibrillator may fail to effect the heart muscle in the desired manner. WO97/28844 discloses a defibrillation system with a pivoting head.

A need therefore exists for an improved defibrillator paddle design. This need is met by the present invention as described and claimed below.

The present invention relates to a defibrillation system according to claim 1. The defibrillator paddle assembly has a handle and a paddle head. The paddle head contains the conductive surface that is used to contact the muscle tissue of the heart during a defibrillation attempt. The paddle head is connected to the handle by a connection, preferably a flexible connection, allowing the relative orientation between the handle and the paddle head to be adjusted. The flexible connection enables the relative orientation between the handle and the paddle head to be selectively adjusted. Accordingly, a physician can selectively change the orientation of the paddle head in relation to the handle in order to more effectively utilize the paddle assembly during a specific circumstance, thereby making the defibrillator paddle less dangerous and more effective.

The flexible connection between the handle and the paddle head can be adjusted either by manual bending or by utilizing adjustment controls that are present on the handle of the paddle assembly.

Preferred embodiments of the assembly, method, and system of the invention are respectively disclosed in the dependent claims 2-11.

### BRIEF DESCRIPTION OF THE DRAWINGS

For a better understanding of the present invention, reference is made to the following description of exemplary embodiments thereof, considered in conjunction with the accompanying drawings, in which:
FIG. 1 is a perspective view of an exemplary embodiment of a defibrillation system;
FIG. 2 is a cross-sectional view of a first exemplary embodiment of a paddle assembly, shown in a straight orientation;
FIG. 3 is a cross-sectional view of the first exemplary embodiment of a paddle assembly, shown in a bent orientation;
FIG. 4 is a cross-sectional view of a second exemplary embodiment of a paddle assembly;
FIG. 5A and B are perspective views of two variants of a third exemplary embodiment of a paddle assembly;
FIG. 6 is a perspective view of a fourth exemplary embodiment of a paddle assembly;
FIG. 7A-C are perspective views of a fifth embodiment of the invention;
FIG. 8A-C are perspective views of three variants of a sixth embodiment of the invention;
FIG. 9A and B are perspective views of a seventh embodiment of the invention;
FIG. 10 is a perspective view of an eight embodiment of the invention;
FIG. 11A-C are a perspective view, a side view and a frontal view of a ninth embodiment of the invention, respectively;
FIG. 12 are perspective views of a tenth embodiment of the invention; and
FIG. 13-15 are perspective view of three improved embodiments of the invention with a handle with an adjustable length.

### BEST MODE FOR CARRYING OUT THE INVENTION

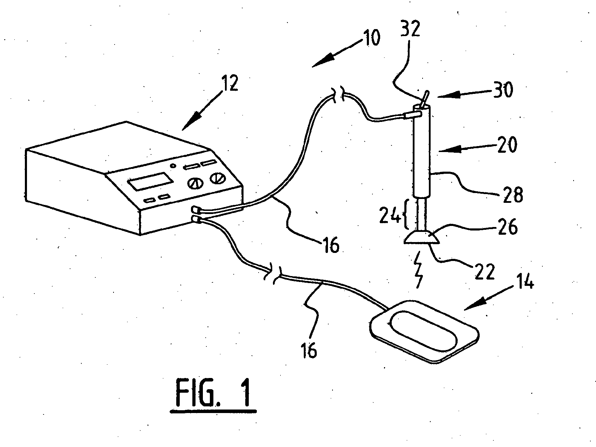

Referring to Fig. 1, there is shown an exemplary embodiment of a defibrillation system 10 in accordance with the present invention. The defibrillation system 10 consists of a control unit 12 that controls the electrical charge that will be passed through the heart muscle. The control unit 12 has adjustable controls and safeguards typical in the industry. There are several defibrillator systems that are commercially available, the control unit of most any prior art defibrillator system can be adapted for use as part of the present invention defibrillation system.

A conductive pad 14 is provided. The conductive pad 14 is connected to the control unit 12 by a flexible terminal wire 16. In use the conductive pad 14 will for example be placed against the back of a patient.

A paddle assembly 20 is also attached to the control unit 12 by a second flexible terminal wire 16. The paddle assembly 20 has a conductive contact surface 22. The control unit 12 creates an electrical bias between the conductive contact surface 22 of the paddle assembly 20 and the conductive pad 14. Accordingly, when both the contact pad 14 and the conductive contract surface 22 of the paddle assembly 20 are touched to a patient, electricity can flow through the patient between these surfaces.

The conductive contact surface 22 is supported by the paddle head 26. The paddle head 26 is set upon a flexible neck 24. The flexible neck 24 attaches the paddle head 26 to a handle 28. The physician using the paddle assembly 20 holds the paddle assembly 20 by grasping the handle 28.

An adjustment control 30 is provided on the handle 28. In the shown embodiment, the adjustment control 30 consists of an adjustment lever 32 that extends out the back of the handle 28. In this position, the adjustment lever 32 can be manipulated by a physician's thumb as the physician grasps the handle 28.

The adjustment control 30 is used to alter the orientation of the paddle head 26 and the conductive contact surface 22 in relation to the handle 28. In the shown embodiment, the conductive contact surface 22 of the paddle head 26 is held in a plane that is generally perpendicular to the longitudinal axis of the handle 28. However, by utilizing the adjustment control 30, the paddle head 26 can be selectively inclined. In this manner, a physician using the paddle assembly 20 can selectively change the orientation of the paddle head 26 and the conductive contact surface 22 so that the conductive contact surface 22 abuts flush against the heart muscle when in use.

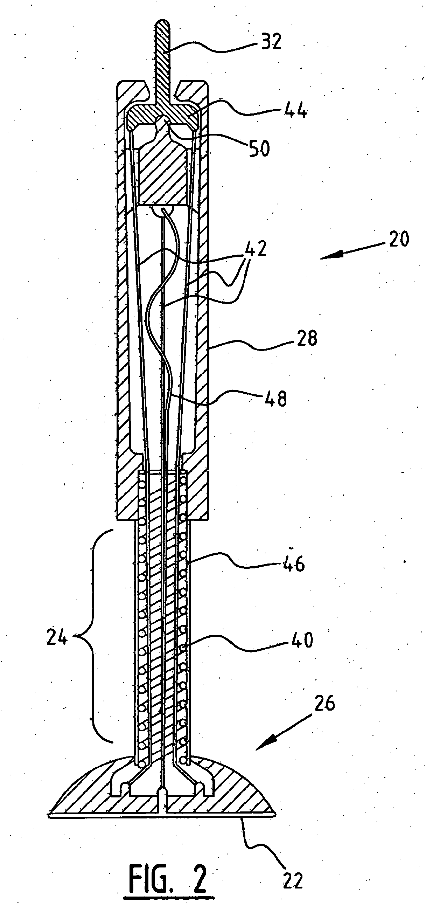

Referring to Fig. 2, it can be seen that a spring 40 is present in the flexible neck 24 of the paddle assembly 20. The spring 40 has one end that attaches to the paddle head 26 and an opposite end that attaches to the handle 28. The spring 40, however, is partially compressed. Accordingly, the spring 40 acts to bias the paddle head 26 away from the handle 28.

The partial compression of the spring 40 is maintained by a plurality of guide wires 42. The guide wires 42 are symmetrically disposed around the inside periphery of the spring 40. Each guide wire 42 has one end that attaches to the paddle head 26. The opposite end of each guide wire 42 attaches to a wobble plate 44 at the bottom of the handle 28. The various guide wires 42 are all held taut by the bias action of the spring 40. The spring 40 also acts as a conduit for the guide wires 42. The guide wires 42 and spring 40 are surrounded by a flexible protective cover 46 in the area of the neck 24 in order to prevent these elements from directly contacting tissue during use of the paddle assembly 20. The terminal wire 48 that provides electricity to the conductive contact surface 22 extends through the handle 28 and through the center of the spring 40 in the flexible neck 24.

The wobble plate 44 is supported by a ball joint 50 at its center. The wobble plate 44 can therefore wobble about the central ball joint 50. The adjustment lever 32 extends away from the wobble plate 44 directly opposite the ball joint 50. Accordingly, it will be understood that by moving the adjustment lever 32 in a particular selected direction, the wobble plate 44 can also be caused to tip toward that selected direction.

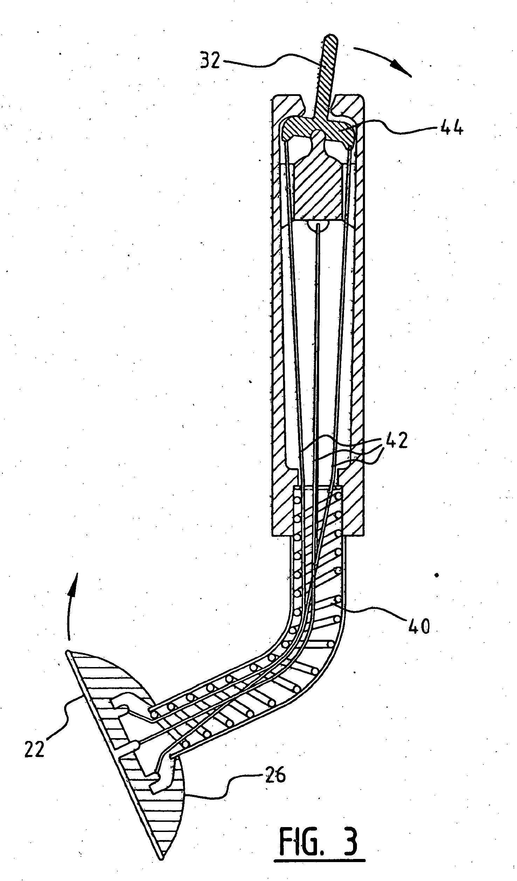

Referring to Fig. 3, it can be seen that when the adjustment lever 32 is moved in a particular direction by a physician, the wobble plate 44 tilts toward that direction. When the wobble plate 44 tilts, the tension in the guide wires 42 at the high side of the wobble plate 44 slacken, while the guide wires 42 attached to the low side of the wobble plate 44 tighten. As the tension in the various guide 42 wires change, the orientation of the paddle head 26 changes. Due to the precompression of the spring 40, as the guide wires 42 on one side of the spring 40 slacken, the spring 40 will expand along that side. Similarly, as the guide wires 42 become more taut on one side of the spring 40, the.spring 40 will compress farther along that side. The result is that the spring 40 will curve away from the slackening guide wires 42 and curve toward the tightening guide wires 42. Preferably, the curvature created in the spring 40 is enough to cause the spring 40 to change orientation by at least ninety degrees. It will be understood that since the adjustment lever 32 can be tilted in any desired direction, the wobble plate 44 can also be tilted in any desired direction. Since the movement of the wobble plate 44 creates a corresponding movement in the paddle head 26, the paddle head 26 can be selectively inclined in any selected direction.

By providing an adjustment mechanism that selectively changes the orientation of the paddle head 26 relative to the paddle handle, a physician can selectively control the orientation of the paddle head 26. The physician can therefore orient the conductive contact surface 22 to lay flush against the heart muscle, regardless of where on the heart muscle the physician decides to touch. This allows the defibrillator paddle to be more affective and present less danger than paddles with a fixed configuration.

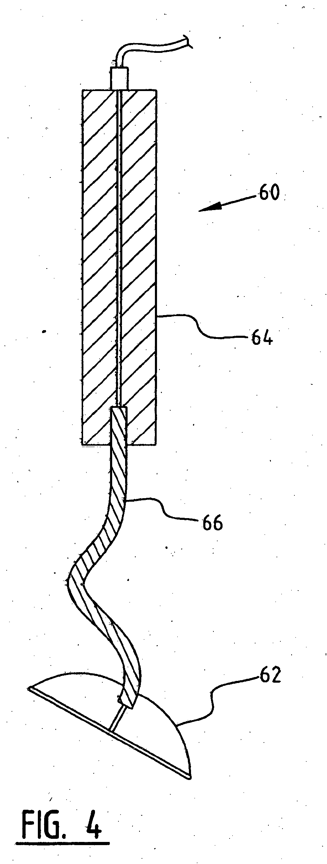

Referring to Fig. 4 an alternate embodiment of the present invention paddle assembly 60 is shown. In this embodiment, a paddle head 62 and a paddle handle 64 are provided. The paddle head 62 and the paddle handle 64 are again coupled together by a flexible neck 66. However, the flexible neck 66 does not contain a spring and guide wires. Rather, the flexible neck 66 of the paddle assembly 60 is fabricated from a section of malleable material, such as a malleable metal. The malleable metal can be a tin alloy, brass alloy or the like that is rigid enough to hold form against gravity, yet malleable enough to be selectively bent into various configurations in the hands of a physician.

The malleable metal in the flexible neck 66 is preferably coated externally with a non-conductive coating 68, such as a dip plastic coating. In this manner, the electricity will not arc from the malleable metal into the surrounding tissue when the paddle assembly 60 is in use.

To use the paddle assembly 60, a physician would grip the paddle head 62 and the paddle handle 64 in opposite hands. The physician would then selectively bend the flexible neck 66 of the paddle assembly 60 until the paddle head 62 is in a desired orientation with respect to the paddle handle 64. The hysician can then use the paddle assembly 60 in its unique orientation to touch the heart muscle.

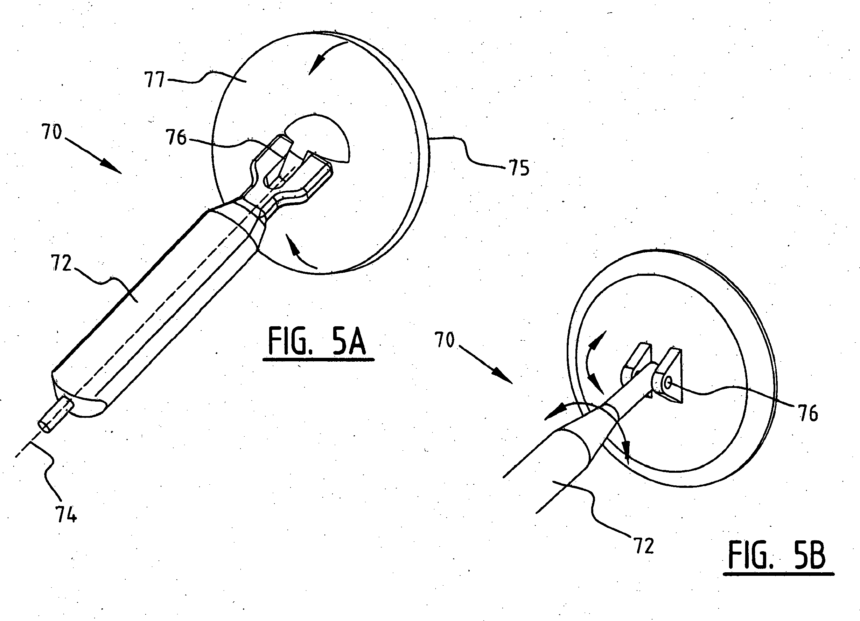

Referring to Fig. 5A, a third-exemplary embodiment of a paddle assembly 70 is shown. In this embodiment, it can be seen that the paddle handle 72 has a longitudinal axis 74 that runs down the center of the paddle handle 72. The paddle handle 72 attaches to a paddle head 77 at a hinged joint 76. The paddle head 77 has a flat contact surface 75. The hinged joint 76 enables the orientation of the flat contact surface 75 to be selectively altered relative to the paddle handle 72. Preferably, the hinged joint 76 enables the paddle head 77 to move at least from a first orientation that is generally parallel to the longitudinal axis 74 of the paddle handle 72 to a second orientation that is generally perpendicular to the longitudinal axis 74 of the paddle handle 72. An internal spring (not shown) may be provided at the hinged joint to bias the paddle handle 72 into one orientation. However, the bias is readily overcome when the paddle head 77 is brought into contact with the tissue of the heart.

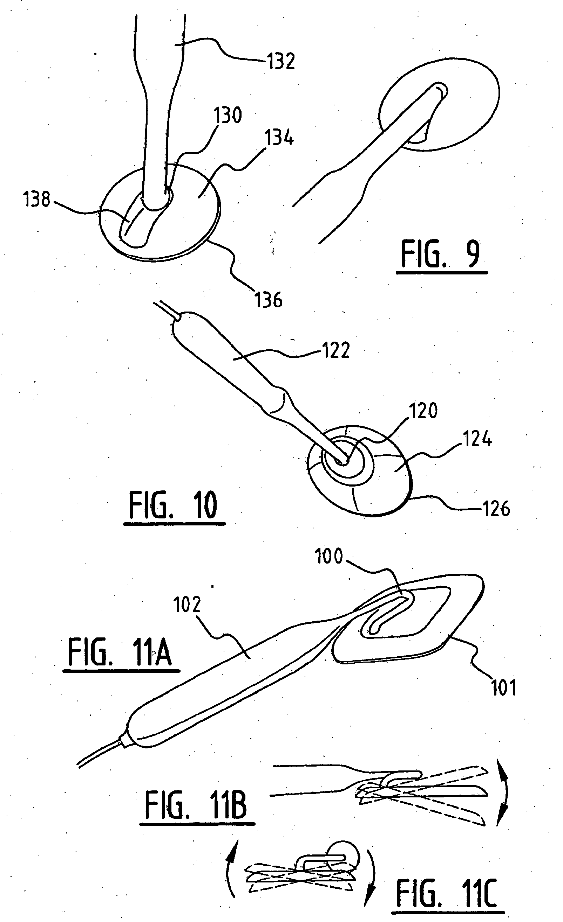

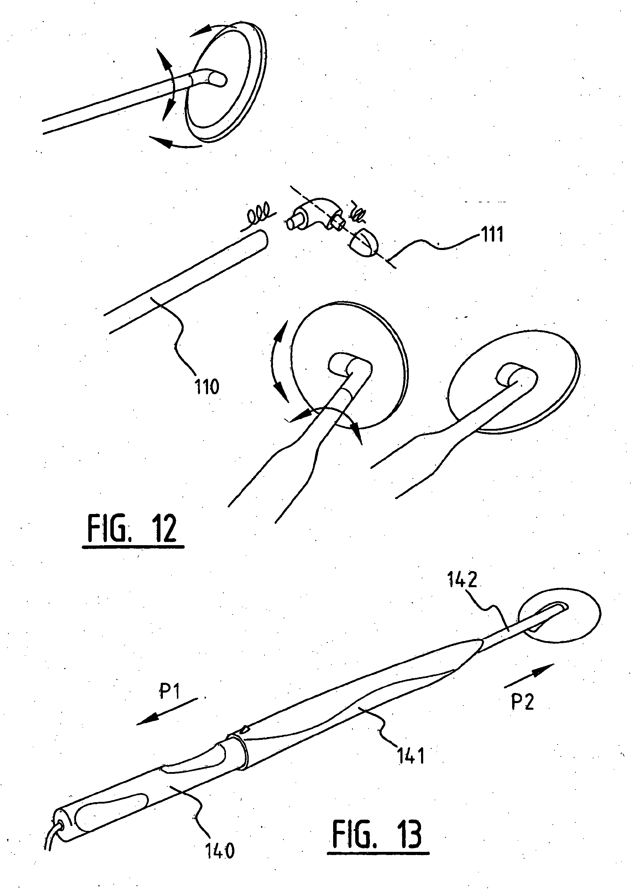

In the embodiment of Fig. 5A, the hinged joint 76 is a simple pin hinge. It will be understood that other types of hinged joints, such as ball and socket joints and universal joints can also be used to attach the paddle handle 72 to the paddle head 77. All such joint configurations are intended to be included within the scope of the claims. Fig. 5B shows an example with a hinge 76 similar to the hinge of fig. 5A, which hinge is built to further allow a limited rotation around the axis of the handle. Fig. 10 shows a variant with a joint 120 allowing the three dimensional movement of the handle 122 relative to the contact surface 126 of the head 124. Figure 12 shows another variant allowing a rotation around the axis 110 of the handle on the one hand, and around an axis 111 perpendicular thereon, on the other hand, wherein springs are added in the joints.

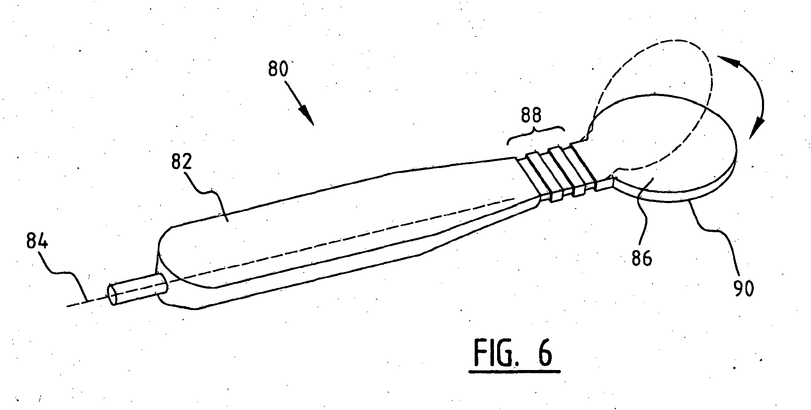

Referring now to Fig. 6, a fourth exemplary embodiment of a paddle assembly 80 is shown. The paddle assembly 80 has a paddle handle 82. The paddle handle 82 runs straight along a longitudinal axis 84. A paddle head 86 is provided. In between the paddle handle 82 and the paddle head 86 is a flexible neck section 88. The flexible neck section 88 can be fabricated by a coated coil spring, but is preferably made from a section of elastic material that is thinned so that it can readily bend.

The paddle head 86 has a flat contact surface 90. The flexible neck section 88 enables the orientation of the flat contact surface 90 to be selectively altered relative to the paddle handle 82. Preferably, the flexible neck section 88 enables the paddle head 86 to move from a first orientation that is generally parallel to the longitudinal axis 84 of the paddle handle 82 to a second orientation that is generally perpendicular to the longitudinal axis 84 of the paddle handle 82.

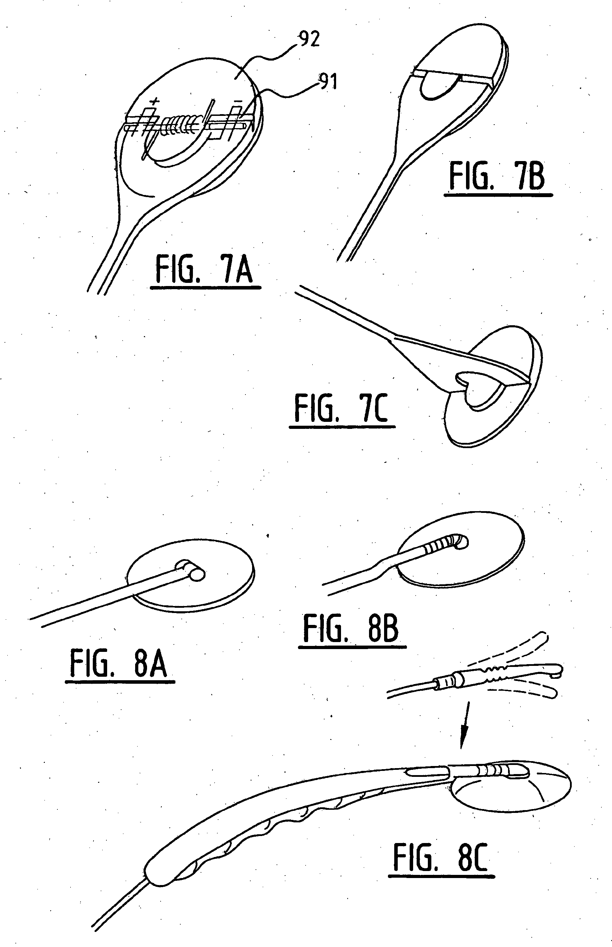

Fig. 7A-C show an embodiment with a hinge 91 which is incorporated in a very flat head 92. Such an embodiment would be especially useful for minimal invasive heart surgery. To allow the paddle assembly to pass through small openings of the body, it is convenient to have a coupling between handle and paddle head, which minimally hinders the movement through such openings. Fig. 8A-C show three other variants of assemblies with a flat head with a connection which extends only over a small distance out of the head. Fig. 9 shows a further embodiment where the connection is located centrally in the head 134, and where a recess 138 is provided in the head for the end part of the handle 132.

Further, fig. 11 shows yet another embodiment which is especially suitable for use in minimal invasive heart surgery. Here the paddle head 101 is substantially lozenge-shaped. Such a form may facilitate the entry of the paddle head 101 in a cut or incision. The skilled person will understand that other forms are possible to improve the insertion of the paddle assembly in cut. As illustrated in fig. 11B and C, respectively, the flexible U-shaped end part 100 of the handle 102 allows on the one hand a rotation of the head around an axis perpendicular to the handle 102, and on the other hand a rotation around the longitudinal axis of the handle 102.

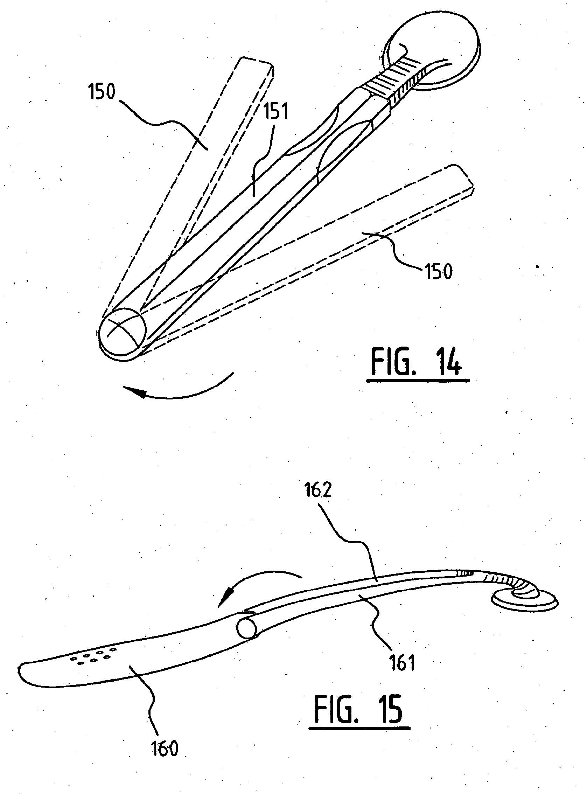

Finally it is pointed out the handle may be telescopic, so that the length of the handle can be adapted depending of the type of operation. Such an embodiment is shown in Fig. 13. Here the handle comprises three parts 140, 141, 142 which are slide into each other - see arrows P1 and P2 - to form a telescopic handle with an adjustable length. In a preferred embodiment the handle is built in such a way that the length is adaptable during the operation. Of course there exist other possibilities to make the handle extendable. Figure 14 shows an embodiment with a handle consisting of a first part 151 and an end part 150, wherein the end part is rotatably connected to part 151 between a position in which part 150, 151 substantially overlap and a position in which part 150 forms an extension of part 151. Figure 15 shows another variant wherein the first part 161 of the handle is provided with a recess 162 in which the end part 160 can be accommodated in the folded position of the handle.

It will be understood that the embodiments of the present invention are merely exemplary and that a person skilled in the art can make many modifications to the shown embodiment using functionally equivalent parts. For instance, in the embodiment of Figs 1-3, the structure of the flexible neck is provided by primarily using a metal spring. In an alternate embodiment, the spring can be replaced with an elastomeric element that would perform the same functions as the spring. Furthermore, the shape and size of the paddle head, neck section and paddle handle are a matter of design choice and can be configured to the whims of the manufacturer. All such variations, modifications and alternate embodiments are intended to be included within the scope of the present invention as set forth in the claims.

## Claims

1. A defibrillation system, comprising:
a control unit (12) for controlling an electric charge between a first terminal and a second terminal;
a conductive pad (14) coupled to said first terminal by a first wire, said conductive pad being adapted for laying against the back of a patient;
a paddle assembly coupled to the second terminal comprising:
a handle (72);
a paddle head (77) having a conductive surface (75) thereon;
a connection (76) coupling said handle to said paddle head; said connection being adapted to enable said paddle head, whilst in contact with the heart, to move through a range of different orientations with respect to said handle, said connection allowing rotation of the paddle head around an axis perpendicular to the handle;
wherein said connection is a flexible connection which includes a spring (40) that extends between said handle and said paddle head.

2. Assembly according to claim 1, further including an adjustment mechanism (30) disposed on said handle for selectively moving said paddle head through said range of different orientations.

3. The assembly according to claim 1 or 2, wherein said spring is in a compressed state.

4. The assembly according to any of the previous claims, further including guide wires (42) that extend between said paddle head and said handle, wherein said guide wires maintain said spring in said compressed state.

5. The assembly according to claim 4, wherein said spring provides tautness to said guide wires and said assembly further includes an adjustment mechanism for selectively varying said tautness of said guide wires.

6. The assembly according to claim 4, further including a wobble plate (44) disposed within said handle, wherein said guide wires attach to said wobble plate.

7. The assembly according to claim 6, further including a lever (32) coupled to said wobble plate for manually manipulating said wobble plate.

8. The assembly according to any of the previous claims, wherein said connection includes a hinge joint (76) that joins said handle to said paddle head.

9. The assembly according to any of the previous claims, wherein said flexible connection includes a length of elastomeric material.

10. The assembly according to any of the previous claims, wherein said flexible connection includes a length of malleable metal.

11. The assembly according to any of the previous claims, **characterized in that** the length of the handle is adjustable.

## Patentansprüche

1. Defibrillationsanlage, umfassend:
eine Steuereinheit (12) zum Steuern einer elektrischen Ladung zwischen einem ersten Anschluss und einem zweiten Anschluss,
ein leitendes Kissen (14), welches durch einen ersten Leiter mit dem ersten Anschluss verbunden ist, wobei das leitende Kissen geeignet ist für ein Anlegen am Rücken eines Patienten,
eine mit dem zweiten Anschluss verbundene Paddle-Baueinheit, umfassend:
einen Griff (72),
einen Paddle-Kopf (77) mit einer leitenden Oberfläche (75) darauf,
eine Verbindung (76), welche den Griff mit dem Paddle-Kopf verbindet,
wobei die Verbindung geeignet ist, dem Paddle-Kopf zu ermöglichen, während dieser mit dem Herzen in Kontakt ist, sich über einen Bereich unterschiedlicher Ausrichtungen in Bezug auf den Griff zu bewegen,
wobei die Verbindung eine Drehung des Paddle-Kopfes um eine Achse rechtwinklig zu dem Griff erlaubt
und wobei die Verbindung eine flexible Verbindung ist, die eine Feder (40) enthält, welche sich zwischen dem Griff und dem Paddle-Kopf erstreckt.

2. Baueinheit nach Anspruch 1, ferner enthaltend einen Einstellmechanismus (30), der an dem Griff zur selektiven Bewegung des Paddle-Kopfes über den genannten Bereich unterschiedlicher Ausrichtungen angeordnet ist.

3. Baueinheit nach Anspruch 1 oder 2, wobei die Feder sich in einem zusammengedrückten Zustand befindet.

4. Baueinheit nach einem der vorhergehenden Ansprüche, ferner enthaltend Führungsdrähte (42), die sich zwischen dem Paddle-Kopf und dem Griff erstrecken, wobei die Führungsdrähte die Feder in dem zusammengedrückten Zustand halten.

5. Baueinheit nach Anspruch 4, wobei die Feder den Führungsdrähten Straffheit verleiht und wobei die Baueinheit ferner einen Einstellmechanismus zum selektiven Verändern der Straffheit der Führungsdrähte enthält.

6. Baueinheit nach Anspruch 4, ferner enthaltend eine Taumelscheibe (44), die in dem Griff angeordnet ist, wobei die Führungsdrähte an der Taumelscheibe hängen.

7. Baueinheit nach Anspruch 6, ferner enthaltend einen Hebel (32), der mit der Taumelscheibe zum manuellen Betätigen der Taumelscheibe gekoppelt ist.

8. Baueinheit nach einem der vorhergehenden Ansprüche, wobei die Verbindung eine Gelenkverbindung (76) enthält, welche den Griff mit dem Paddle-Kopf verbindet.

9. Baueinheit nach einem der vorhergehenden Ansprüche, wobei die flexible Verbindung ein Stück elastomeres Metall enthält.

10. Baueinheit nach einem der vorhergehenden Ansprüche, wobei die flexible Verbindung ein Stück dehnbares Metall enthält.

11. Baueinheit nach einem der vorhergehenden Ansprüche, **dadurch gekennzeichnet, dass** die Länge des Griffes einstellbar ist.

## Revendications

1. Système de défibrillation, comprenant :
une unité de commande (12) pour commander une charge électrique entre un premier terminal et un second terminal ;
une pastille conductrice (14) couplée audit premier terminal par un premier câble, ladite pastille conductrice étant adaptée pour être posée contre le dos d'un patient ;
un ensemble électrode couplé au second terminal comprenant :
une poignée (72) ;
une tête d'électrode (77) ayant une surface conductrice (75) sur celle-ci ;
un raccord (76) couplant ladite poignée à ladite tête d'électrode ; ledit raccord étant adapté pour permettre à ladite tête d'électrode, pendant que celle-ci est en contact avec le coeur, de se déplacer à travers une plage d'orientations différentes relativement à ladite poignée, ledit raccord permettant la rotation de la tête d'électrode autour d'un axe perpendiculaire à la poignée ; dans lequel ledit raccord est un raccord flexible qui comprend un ressort (40) qui s'étend entre ladite poignée et ladite tête d'électrode.

2. Ensemble selon la revendication 1, incluant en outre un mécanisme d'ajustement (30) disposé sur ladite poignée pour sélectivement déplacer ladite tête d'électrode à travers ladite plage d'orientations différentes.

3. Ensemble selon la revendication 1 ou 2, dans lequel ledit ressort est dans un état comprimé.

4. Ensemble selon l'une quelconque des revendications précédentes, incluant en outre des câbles-guide (42) qui s'étendent entre ladite tête d'électrode et ladite poignée, dans lequel lesdits câbles-guides maintiennent ledit ressort dans ledit état comprimé.

5. Ensemble selon la revendication 4, dans lequel ledit ressort fournit une tension auxdits câbles-guides et ledit ensemble comprend en outre un mécanisme d'ajustement pour sélectivement faire varier la tension desdits câbles-guides.

6. Ensemble selon la revendication 4, incluant en outre une plaque oscillante (44) disposée à l'intérieur de ladite poignée, dans lequel lesdits câbles-guides se fixent à ladite plaque oscillante.

7. Ensemble selon la revendication 6, incluant en outre un levier (32) couplé à ladite plaque oscillante pour manuellement manipuler ladite plaque oscillante.

8. Ensemble selon l'une quelconque des revendications précédentes, dans lequel ledit raccord comprend une articulation à charnière (76) qui articule ladite poignée à ladite tête d'électrode.

9. Ensemble selon l'une quelconque des revendications précédentes, dans lequel ledit raccord flexible inclut une longueur de matériau élastomère.

10. Ensemble selon l'une quelconque des revendications précédentes, dans lequel ledit raccord flexible inclut une longueur de métal malléable.

11. Ensemble selon l'une quelconque des revendications précédentes, **caractérisé en ce que** la longueur de la poignée est ajustable.
